# EUROPEAN PATENT APPLICATION

(11) **EP 4 534 206 A1**
(43) Date of publication of application: **09.04.2025**
(21) Application number: 23812131.3
(22) Date of filing: 24.05.2023
(51) Int. Cl.: B01L 7/00, C12Q 1/686

(54) **TEMPERATURE CONTROL MODULE FOR PCR DEVICE AND PCR DEVICE HAVING SAME**

(30) Priority: 24.05.2022 KR 20220063178
(71) Applicant: Optolane Technologies Inc., Bundang-gu Seongnam-si, Gyeonggi-do 13494 (KR)
(72) Inventor: CHO, Seung Sun, Ansan-si Gyeonggi-do 15524 (KR); SONG, Keun Hyung, Seoul 05054 (KR); YOON, Chang Hwan, Seoul 02588 (KR); LEE, Do Young, Seoul 06002 (KR)
(74) Representative: Schiweck Weinzierl Koch Patentanwälte Partnerschaft mbB
(86) International application number: PCT/KR2023/007045
(87) International publication number: WO 2023/229357

(57) **Abstract**

Disclosed are a temperature control module for a PCR device and a PCR device having same, the temperature control module being capable of more rapidly increasing or decreasing the temperature of a sample unit. The temperature control module for a PCR device comprises: a motor; a shaft assembly comprising a shaft connected to the rotary axis of the motor so as to move up and down in accordance with the rotation of the motor; a Peltier element placed on the upper portion of the shaft assembly; and a temperature-modifying chuck which, in an initial mode, receives temperature-modifying heat from the Peltier element and, in a temperature-control mode, moves upward on the shaft to supply temperature-modifying heat to a temperature-control surface of a PCR cartridge. Accordingly, the temperature of the sample unit can increase or decrease more rapidly by having the temperature-modifying chuck placed in the temperature control module for a PCR device, wherein the temperature-modifying chuck receives temperature-modifying heat from the Peltier element for heating or cooling in the initial mode and moves upward on the shaft to supply temperature-modifying heat to a temperature-control surface of a PCR cartridge in a temperature-control mode.

## Description

### Technical Field

The present application claims the benefit of priority based on Korean Patent Application No. 10-2022-0063178 filed on May 24, 2022, the entire disclosure of which is incorporated as a part of this specification.

The present disclosure relates to a temperature control module for a PCR device and a PCR device having the same, and more specifically, to a temperature control module for a PCR device capable of more quickly raising or lowering the temperature of a sample unit and a PCR device having the same.

### Background Art

In general, genetic diagnosis is performed by extracting deoxyribonucleic acid (hereinafter referred to as "DNA") from a small amount of sample, such as body fluid, blood, or somatic cells, and then performing a DNA test. At this time, most of the extracted DNA is in a small amount and low purity.

In order to increase the accuracy of genetic diagnosis, it is necessary to amplify the amount of DNA extracted from the sample, or to amplify only a specific part of the base sequence of the extracted DNA. For example, polymerase chain reaction (hereinafter referred to as "PCR") is known as a technology that amplifies only specific DNA and specific base sequences within DNA as a preliminary step for using genetic diagnosis technology.

In other words, this PCR is a technology that repeatedly heats and cools a sample solution containing nucleic acids to serially replicate a part having a specific base sequence of the nucleic acid and exponentially amplifies the nucleic acid having that specific base sequence part, and is widely used in the fields of life science, genetic engineering, and medicine.

The PCR generally achieves DNA amplification through a denaturation step, annealing step, and extension step. The PCR is performed by a PCR device.

The PCR device uses a temperature controller to control the temperature of the sample unit to a temperature for each step required for DNA synthesis. Since the temperature of the sample unit is controlled by the temperature controller, there may be a delay in raising or lowering the temperature of the sample unit, and thus it may take a long time to complete the PCR.

Therefore, there is a need to raise or lower the temperature of the sample unit more quickly.

### Disclosure of the Invention

### Technical Goals

Accordingly, the technical problem of the present disclosure was conceived in this regard, and an object of the present disclosure is to provide a temperature control module for a PCR device capable of raising or lowering the temperature of the sample unit more quickly.

Another object of the present disclosure is to provide a PCR device having the temperature control module for a PCR device.

### Technical Solutions

In order to realize the above object of the present disclosure, a temperature control module for a PCR device according to an embodiment of the present disclosure includes a motor; a shaft assembly comprising a shaft connected to a rotary axis of the motor so as to move up and down in accordance with a rotation of the motor; a Peltier element disposed above the shaft assembly; and a temperature-modifying chuck configured to, in an initial mode, receive temperature-modifying heat from the Peltier element and, in a temperature control mode, move upward on the shaft to supply temperature-modifying heat to a temperature control surface of a PCR cartridge.

### Advantageous Effects

According to the temperature control module for a PCR device and the PCR device having the same, the temperature-modifying chuck configured to, in the initial mode, receive temperature-modifying heat, such as cooling heat or heating heat from the Peltier element, and in the temperature control mode, move upward on the shaft to supply temperature-modifying heat to the temperature control surface of the PCR cartridge is disposed in the temperature control module for the PCR device, thereby enabling the temperature of the sample unit to be raised or lowered more quickly.

### Brief Description of Drawings

FIG. 1 is a perspective view for explaining a temperature control module for a PCR device according to an embodiment of the present disclosure.
FIG. 2 is a perspective view for explaining that a PCR cartridge is disposed on a temperature control module for a PCR device illustrated in FIG. 1.
FIG. 3A is a front perspective view for explaining a PCR cartridge illustrated in FIG. 2, and FIG. 3B is a back perspective view for explaining a PCR cartridge illustrated in FIG. 2.
FIG. 4A is a photograph taken from a side direction of a temperature control module for a PCR device of the present disclosure implemented as a product, FIG. 4B is a photograph taken from an oblique direction of a temperature control module for a PCR device of FIG. 4A, and FIG. 4C is a photograph taken from an oblique direction in which a PCR cartridge is disposed on a temperature control module for a PCR device of FIG. 4A for temperature control.
FIG. 5 is a perspective view for explaining a shaft assembly illustrated in FIG. 1.
FIG. 6 is a perspective view for explaining a lower chuck, a compression spring, and an upper chuck illustrated in FIG. 1.
FIG. 7 is a cross-sectional view for explaining an initial mode between a temperature control module for a PCR device illustrated in FIG. 1 and a PCR cartridge.
FIG. 8 is a cross-sectional view for explaining a temperature control mode between a temperature control module for a PCR device illustrated in FIG. 1 and a PCR cartridge.
FIG. 9 is a block diagram for schematically explaining a PCR device according to an embodiment of the present disclosure.

### Best Mode for Carrying Out the Invention

In order to achieve the above object of the present disclosure, a temperature control module for a PCR device according to an embodiment of the present disclosure includes a motor; a shaft assembly comprising a shaft connected to a rotary axis of the motor so as to move up and down in accordance with a rotation of the motor; a Peltier element disposed above the shaft assembly; and a temperature-modifying chuck configured to, in an initial mode, receive temperature-modifying heat from the Peltier element and, in a temperature control mode, move upward on the shaft to supply temperature-modifying heat to a temperature control surface of a PCR cartridge.

In an embodiment, the temperature-modifying chuck may include a lower chuck having a first area covering the Peltier element; and an upper chuck having a second area formed to contact the temperature control surface of the PCR cartridge. Here, a first groove may be formed on an upper surface of the lower chuck, and a second groove facing the first groove may be formed on a lower surface of the upper chuck.

In an embodiment, the temperature control module may further include a compression spring chuck disposed in the first groove and the second groove, and configured to perform a buffering function when the upper chuck contacts the temperature control surface of the PCR cartridge.

In an embodiment, the temperature control module may further include a heat sink assembly in contact with a heat generating portion of the Peltier element to increase an efficiency of the Peltier element.

In an embodiment, the heat sink assembly may include one or more heat pipes having one side part in contact with the Peltier element; and a heat sink connected to the other side part of the heat pipes.

In an embodiment, the temperature control module may further include a cooling fan disposed adjacent to the heat sink to cool heat of the heat sink.

In order to achieve another object of the present disclosure, a PCR device according to an embodiment of the present disclosure includes a PCR module; and a temperature control module in contact with the PCR module to raise or lower a temperature of the PCR module, wherein the temperature control module includes a motor; a shaft assembly comprising a shaft connected to a rotary axis of the motor so as to move up and down in accordance with a rotation of the motor; a Peltier element disposed above the shaft assembly; and a temperature-modifying chuck configured to, in an initial mode, receive temperature-modifying heat from the Peltier element and, in a temperature control mode, move upward on the shaft to supply temperature-modifying heat to a temperature control surface of a PCR cartridge.

### Modes for Carrying Out the Invention

Hereinafter, the present disclosure will be described in more detail with reference to the attached drawings. Since the present disclosure may be variously modified and may have various forms, specific embodiments will be illustrated in the drawings and described in detail in the detailed description. However, this is not intended to limit the present disclosure to a specific disclosed form, and it should be understood that all modifications, equivalents, and substitutes are included in the spirit and scope of the present disclosure.

In describing each drawing, similar reference numerals are used for similar components. In the attached drawings, the dimensions of structures are enlarged from the actual sizes for clarity of the present disclosure.

Terms such as first and second may be used to describe various components, but the components should not be limited by the terms. The terms are used only for the purpose of distinguishing one component from another. For example, a first component may be referred to as a second component without departing from the scope of the present disclosure, and similarly, the second component may also be referred to as the first component. Singular expressions include plural expressions unless the context clearly means otherwise.

In this specification, it should be understood that terms such as "include" or "have" are intended to designate the presence of features, numbers, steps, operations, components, parts, or combinations thereof described in the specification, but do not preclude the possibility of the presence or addition of one or more other features, numbers, steps, operations, components, parts, or combinations thereof.

In addition, unless otherwise defined, all terms used herein, including technical or scientific terms, have the same meaning as those generally understood by a person skilled in the art to which the present disclosure pertains. Terms defined in dictionaries generally used should be construed to have meanings matching contextual meanings in the related art and are not to be construed as an ideal or excessively formal meaning unless otherwise defined herein.

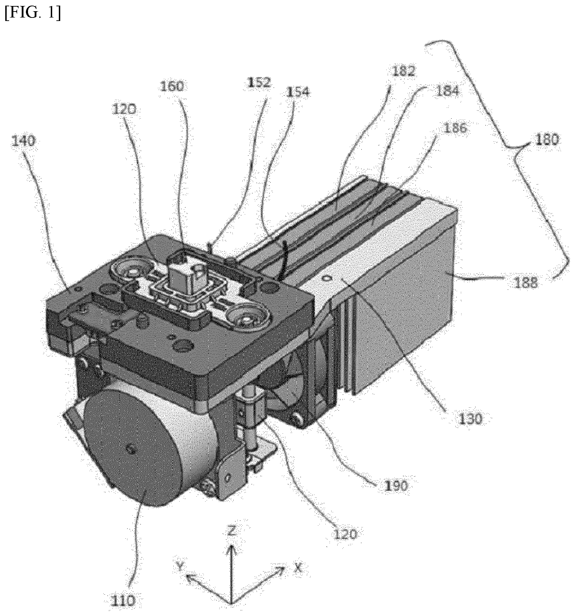

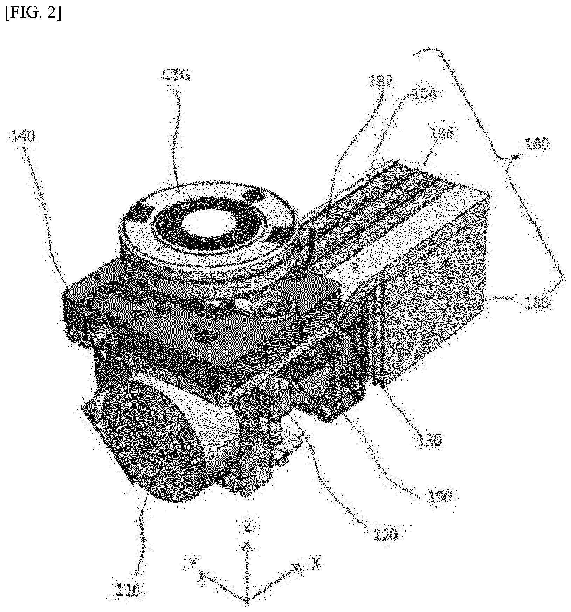

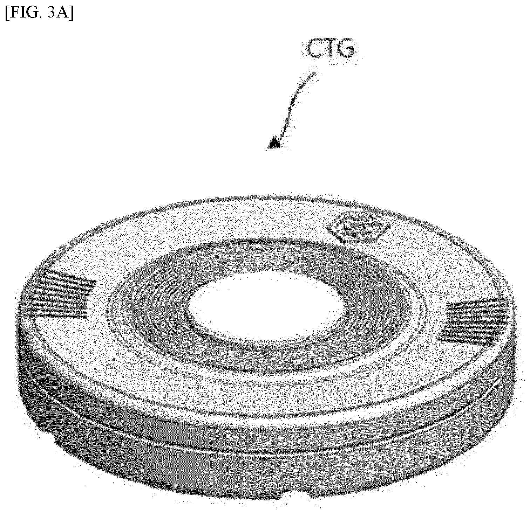

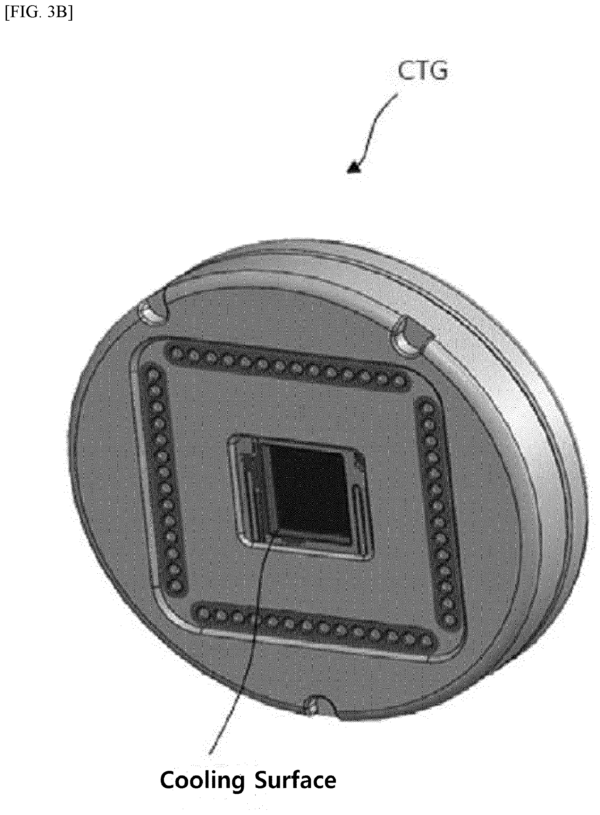

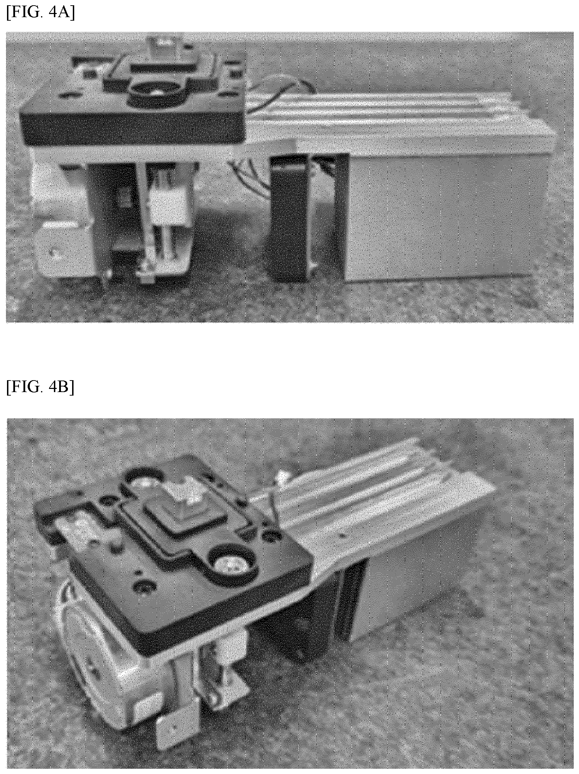

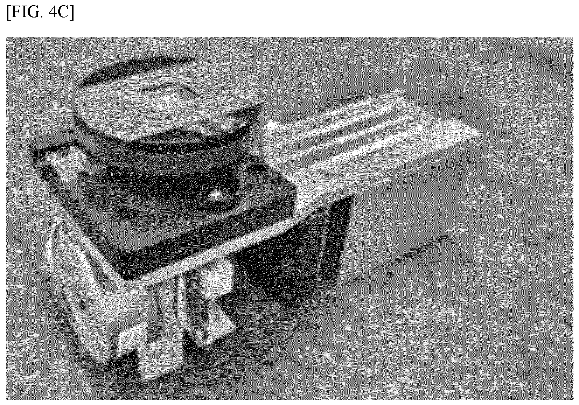

FIG. 1 is a perspective view for explaining a temperature control module for a PCR device according to an embodiment of the present disclosure. FIG. 2 is a perspective view for explaining that a PCR cartridge is disposed on the temperature control module for a PCR device illustrated in FIG. 1. FIG. 3A is a front perspective view for explaining the PCR cartridge illustrated in FIG. 2, and FIG. 3B is a back perspective view for explaining the PCR cartridge illustrated in FIG. 2. FIG. 4A is a photograph taken from a side direction of a temperature control module for a PCR device of the present disclosure implemented as a product, FIG. 4B is a photograph taken from an oblique direction of the temperature control module for a PCR device of FIG. 4A, and FIG. 4C is a photograph taken from an oblique direction in which a PCR cartridge is disposed on the temperature control module for a PCR device of FIG. 4A for temperature control.

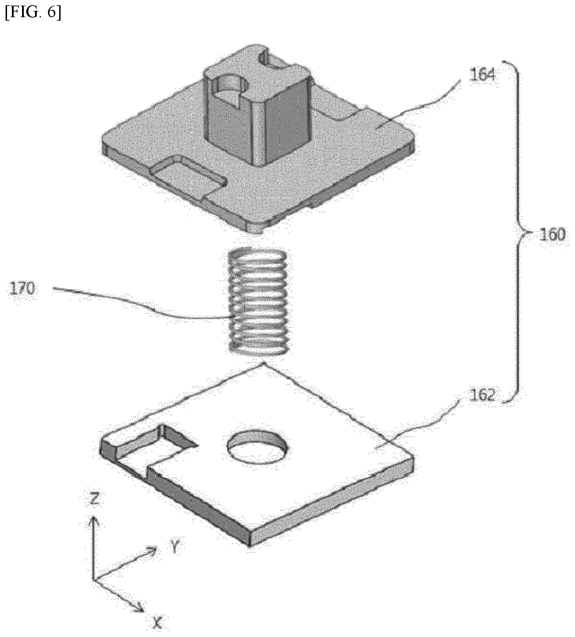

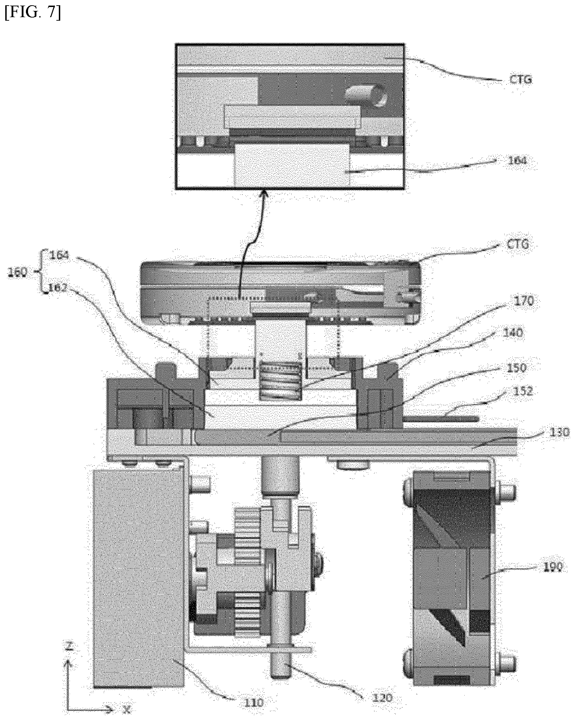

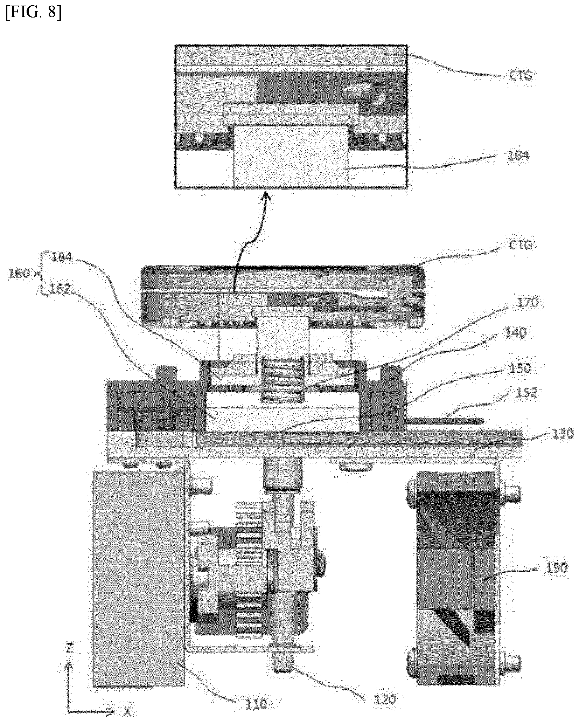

Referring to FIGS. 1 to 4C, a temperature control module for a PCR device according to an embodiment of the present disclosure includes a motor 110, a shaft assembly 120, a first support member 130, a second support member 140, a Peltier element 150 (illustrated in FIGS. 7 and 8), a temperature-modifying chuck 160, a compression spring chuck 170 (illustrated in FIG. 6), a heat sink assembly 180, and a cooling fan 190.

The motor 110 is disposed below the first support member 130 and is fastened to the first support member 130 via a bracket.

The shaft assembly 120 is connected to the rotary axis of the motor 110 and moves the temperature-modifying chuck 160 between the Peltier element 150 and the PCR cartridge (CTG) in accordance with the rotation of the motor 110.

The first support member 130 is disposed on the XY plane and has a plate shape extending along the X-axis. In a portion below the first support member 130, the motor 110 connected via the bracket is disposed. In another portion below the first support member 130, the heat sink assembly 180 and the cooling fan 190 are disposed. In still another portion below the first support member 130, the shaft assembly 120 is disposed. In the first support member 130, a hole is formed so that the shaft assembly 120 may pass upward.

The second support member 140 has a square shape and is fastened on the first support member 130 while surrounding the Peltier element 150. The central region of the second support member 140 is opened to expose the temperature-modifying chuck 160 and has an upper region in the shape of a cage so that the temperature-modifying chuck 160 is not completely separated to the outside.

The Peltier element 150 is disposed above the first support member 130 and disposed above the shaft assembly 120 disposed below the first support member 130. The Peltier element 150 is disposed below the temperature-modifying chuck 160 to correspond to the region in which the central region of the second support member 140 is opened. The Peltier element 150 is configured to utilize various effects resulting from the interaction of heat and electricity. For example, the Peltier element 150 may utilize the Peltier effect of absorbing or releasing heat depending on the direction of the current applied through the first wire 152 and the second wire 154. The Peltier element 150 is configured internally with an n-type semiconductor element and a p-type semiconductor element, and is configured by disposing bonding metal plates on both side surfaces thereof, and is inserted into a hole formed in a guide member. When power is supplied to the Peltier element 150 through the first wire 152 and the second wire 154, an absorption reaction occurs on one side surface of the two bonding metal plates, and a heat dissipation reaction that releases the absorbed heat occurs on the other side surface. When the direction in which the current supplied through the first wire 152 and the second wire 154 flows is changed, the flow of electrons and holes also changes, so that an absorption reaction occurs on the other side surface of the two bonding metal plates, and a heat dissipation reaction that releases the absorbed heat occurs on one side surface. If a heat exchanger is formed on the heat absorption surface of the Peltier element 150 having this principle, the ambient temperature of the heat absorption surface may be cooled, and if a heat exchanger is formed on the heat dissipation surface, the ambient temperature may be raised. As the area of the Peltier element 150 becomes larger, the cooling power by the cooling heat or the heating power by the heating heat may become larger.

The temperature-modifying chuck 160 receives temperature-modifying heat such as cooling heat or heating heat from the Peltier element in the initial mode, and in the temperature control mode, moves upward on the shaft to supply temperature-modifying heat to the temperature control surface of the PCR cartridge. When temperature-modifying heat is supplied to the temperature control surface of the PCR cartridge, the sample unit in the PCR cartridge is cooled or heated.

The compression spring chuck (not shown) performs a buffering function when the upper chuck 164 comes into contact with the temperature control surface of the PCR cartridge (CTG). A detailed description of the compression spring chuck will be described later in FIG. 6.

The heat sink assembly 180 includes first to third heat pipes 182, 184, and 186 having one side part in contact with the Peltier element 150 and a heat sink 188 connected to the other side part of each of the first to third heat pipes 182, 184, and 186. The first to third heat pipes 182, 184, and 186 have a flat structure so that a portion of the upper surface thereof may smoothly contact the Peltier element 150. The first to third heat pipes 182, 184, and 186 are based on the principle that when a liquid such as water or alcohol is put inside a depressurized pipe and one side is heated, the liquid turns into vapor and flows to the other side, and when the heat is dissipated there and turns into liquid, the liquid returns to the heating part by capillary action. Due to the repetition of this action, the heat of the Peltier element 150 is transferred to the heat sink 188. The first to third heat pipes 182, 184, and 186 are inserted into each of the grooves formed in the first support member 130. The first to third heat pipes 182, 184, and 186 may have a circular cross-section or a rounded rectangular cross-section. The heat sink 188 absorbs heat provided from the first to third heat pipes 182, 184, and 186 and releases the heat to the outside using the airflow of the cooling fan 190. The heat sink 188 is formed with a plurality of fins so that it may receive heat from the first to third heat pipes 182, 184, and 186 and evenly distribute the heat throughout the entire heat sink 188, and the wind blowing from the cooling fan 190 may easily escape, so that heat generation may easily occur.

The cooling fan 190 cools the heat sink 188 by forcibly flowing air. The cooling fan 190 may forcibly suck in air through a penetration part provided in the fan bracket and cool the heat sink assembly 180 in which high temperature heat has accumulated by flowing the air. The cooling fan 190 may be connected to the fan bracket with screws or by bolts.

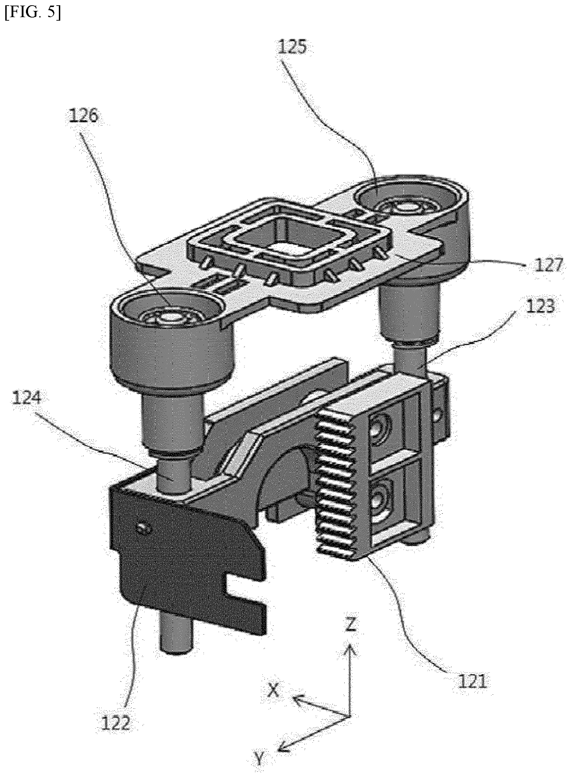

FIG. 5 is a perspective view for explaining a shaft assembly illustrated in FIG. 1.

Referring to FIGS. 1 to 5, the shaft assembly 120 includes a rack gear 121, a sensor actuator 122, a first shaft 123, a second shaft 124, a first spring damper chuck 125, a second spring damper chuck 126, and a temperature-modifying chuck guide member 127, and is connected to the rotary axis of the motor 110 to move the temperature-modifying chuck 160 between the Peltier element 150 and the PCR cartridge (CTG) in accordance with the rotation of the motor 110.

The rack gear 121 moves up and down (in the Z-axis direction or -Z-axis direction) in conjunction with the rotary axis of the motor 110 to move the location of the sensor actuator 122 up and down.

The sensor actuator 122 is fastened to the rack gear 121 and is interlocked according to the up and down movement of the rack gear 121. The chuck location sensor is disposed to be fixed near the motor 110 side to detect the rising or descending of the sensor actuator 122. When the sensor actuator 122 is detected to be descending, the chuck location sensor determines that it is in contact with the Peltier element. On the other hand, when the sensor actuator 122 is detected to be rising, the chuck location sensor determines that it is separated from the Peltier element and in contact with the temperature control surface of the PCR cartridge.

The first shaft 123 moves up and down (in the Z-axis direction or the -Z-axis direction), and the second shaft 124 moves up and down (in the Z-axis direction or the -Z-axis direction).

The first spring damper chuck 125 is disposed at the upper end part of the first shaft 123, and the second spring damper chuck 126 is disposed at the upper end part of the second shaft 124.

The temperature-modifying chuck guide member 127 is assembled between the first spring damper chuck 125 and the second spring damper chuck 126 to prevent the temperature-modifying chuck 160 from tilting.

FIG. 6 is a perspective view for explaining a lower chuck, a compression spring, and an upper chuck illustrated in FIG. 1.

Referring to FIGS. 1 to 6, the temperature-modifying chuck 160 includes a lower chuck 162 having a first area covering the Peltier element 150 and an upper chuck 164 having a second area formed to contact the temperature control surface of the PCR cartridge (CTG), and supplies temperature-modifying heat of the Peltier element 150 to the temperature control surface of the PCR cartridge (CTG). In other words, the lower chuck 162, in the initial mode, is disposed adjacent to the Peltier element 150 and receives temperature-modifying heat from the Peltier element 150. In addition, the lower chuck 162, in the temperature control mode, moves upward along with the upward movement of the shaft so as to contact the temperature control surface of the PCR cartridge (CTG) and supplies temperature-modifying heat to the temperature control surface of the PCR cartridge (CTG).

A first groove is formed on the upper surface of the lower chuck 162, and a second groove facing the first groove is formed on the lower surface of the upper chuck 164. A compression spring chuck 170 is disposed in the space between the first groove and the second groove.

The compression spring chuck 170 performs a buffering function when the upper chuck 164 comes into contact with the temperature control surface of the PCR cartridge (CTG).

FIG. 7 is a cross-sectional view for explaining an initial mode between a temperature control module for a PCR device illustrated in FIG. 1 and a PCR cartridge. FIG. 8 is a cross-sectional view for explaining a temperature control mode between a temperature control module for a PCR device illustrated in FIG. 1 and a PCR cartridge.

Referring to FIGS. 1 to 8, in the initial mode, the temperature-modifying chuck 160 maintains a state of contacting the Peltier element 150. At this time, the upper surface of the upper chuck 164 is not in contact with any object, i.e., in a free state, the compression spring chuck 170 disposed between the upper chuck 164 and the lower chuck 162 maintains an extended state.

The Peltier element 150 may generate temperature-modifying heat or heating heat depending on the direction of the flowing current. Since the temperature-modifying chuck 160 maintains a state of contacting the Peltier element 150, cooling heat may be supplied or heating heat may be supplied to the temperature-modifying chuck 160. Hereinafter, in the present embodiment, an example in which cooling heat is generated in the Peltier element 150 and cooling heat is supplied to the temperature-modifying chuck 160 will be described.

In the temperature control mode (the mode shown in FIG. 8), the shaft of the shaft assembly 120 moves upward by the rotation of the motor 110. As the shaft moves upward, the temperature-modifying chuck 160 also moves upward, so that the upper chuck 164 of the temperature-modifying chuck 160 contacts the temperature control surface of the PCR cartridge (CTG) and supplies cooling heat to the PCR cartridge (CTG).

In the present embodiment, the compression spring chuck 170 performs a buffering function when the upper chuck 164 contacts the temperature control surface of the PCR cartridge (CTG). In other words, when the shaft moves upward (i.e., in the Z-axis direction) in accordance with the operation of the shaft assembly 120, the temperature-modifying chuck 160 disposed above the shaft assembly 120 also moves upward.

When the temperature-modifying chuck 160 moving upward comes into contact with the temperature control surface of the PCR cartridge (CTG), the compression spring chuck 170 disposed inside the temperature-modifying chuck 160, i.e., between the upper chuck 164 and the lower chuck 162, is switched to a compressed state. Accordingly, it is possible to prevent the temperature-modifying chuck 160 from being moved upward forcibly, thereby causing scratches on the temperature control surface of the PCR cartridge (CTG) or applying excessive force.

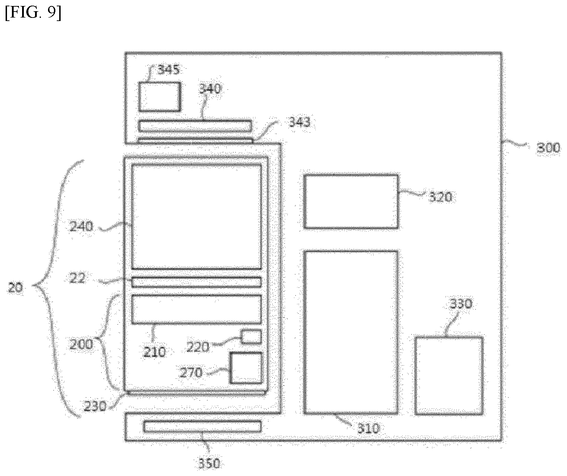

FIG. 9 is a block diagram for schematically explaining a PCR device according to an embodiment of the present disclosure.

Referring to FIG. 9, a PCR device according to an embodiment of the present disclosure includes a PCR module 20 and a reader system 300.

The PCR module 20 includes a wide-angle emission filter 22, an optical sensor substrate 200, a reaction space 240, and a first temperature control module 270. The PCR module 20 includes a wide-angle emission filter 22, an optical sensor substrate 200, a reaction space 240, and a first temperature control module 270. In the present embodiment, the reaction space 240 may be a PCR cartridge (CTG) described in FIGS. 1 to 8, and the first temperature control module 270 may be a temperature control module for a PCR device described in FIGS. 1 to 8.

The wide-angle emission filter 22 is integrally formed on the optical sensor substrate 200, and may block excitation light generated from a light source 340 and transmit emission light generated from a sample in the reaction space 240.

The wide-angle emission filter 22 may include a base medium, a semi-cured photoresist, and a pigment.

The base medium is disposed in a flat shape on the optical sensor substrate 200 to form the outer shape of the wide-angle emission filter 22. The base medium may use a transparent synthetic resin, glass, metal oxide, or the like. In the present embodiment, the base medium may include an epoxy resin, silicone resin, or the like that does not generate fluorescence or phosphorescence and has biocompatible properties.

The semi-cured photoresist is dispersed in the base medium and includes a photoresist that is fixed in a solid state by thermal curing, drying, photocuring, or the like. For example, the semi-cured photoresist may include a negative photoresist or a positive photoresist.

Although not intended to limit the scope of the present disclosure by theory, in order to explain the present disclosure in more detail, the reason why the wide-angle emission filter 22 of the present disclosure has unique excellent optical characteristics is explained as follows.

A general color filter selectively transmits light by fixing a pigment in a transparent medium, thereby absorbing light of a certain wavelength in the pigment and transmitting light of a different wavelength. Since the photoresist has a characteristic that its chemical and optical properties change in response to light with short wavelengths such as ultraviolet light, blue light, and green light, there is a problem that the optical properties change over time when a semi-cured photoresist is used in a color filter. Therefore, a thermosetting material that is completely saturated with light with short wavelengths such as ultraviolet light, blue light, and green light or does not change at all even when irradiated with light with short wavelengths may be used in a conventional color filter.

However, since the wide-angle emission filter 22 of the present disclosure is used in disposable experimental equipment rather than for long-term use, it is not necessary to maintain the same optical characteristics for a long time, and it is only necessary to temporarily maintain the optical characteristics for a relatively short experimental time. Specifically, when a semi-cured photoresist is irradiated with light with a short wavelength such as ultraviolet light, blue light, or green light, it absorbs light with a short wavelength for a certain period of time, so it temporarily functions as an optical filter with very excellent characteristics, but as time passes, it becomes saturated with light with a short wavelength and loses most of its optical filter function, so that a semi-cured photoresist that is contrary to long-term stability could not be used in conventional color filters.

Conversely, the present disclosure utilizes the characteristic of a semi-cured photoresist absorbing light with a short wavelength in the process of becoming saturated with light with a short wavelength such as ultraviolet light, blue light, or green light and becoming stabilized, thereby implementing the wide-angle emission filter 22 with very excellent optical characteristics that may be used in a disposable experimental equipment. In other words, in the present disclosure, by first blocking the excitation light with a pigment and secondarily blocking the excitation light with a semi-cured photoresist, the wide-angle emission filter 22 having excellent characteristics regardless of the direction of incident light, which could not be implemented by existing color filters or interference filters, has been successfully manufactured.

The pigment is a material that absorbs light of a certain wavelength, and for example, yellow pigment, red pigment, blue pigment, green pigment, etc., may be used. In the present embodiment, the pigment includes a yellow pigment. The yellow pigment may include inorganic dyes such as lead chromate, calcium yellow, yellow oxides, complex inorganic colourpigments, bismuth vanadate, etc., or organic dyes such as arylamide, diarylide, benzimidazolone, disazo ondensation, organic metal complexes, isoindoline, quinophthalone, anthrapyrimidine, flavanthrone, etc.

The optical sensor substrate 200 includes a base substrate (not shown), an optical sensor array 210, a temperature sensor 220, and a first temperature control member 230.

The base substrate has a flat shape and is formed integrally with the wide-angle emission filter 22. The base substrate may include various materials such as silicon, plastic, and ceramic.

The optical sensor array 210 is embedded in an upper part of the base substrate so that the upper surface of the base substrate becomes a flat shape. The optical sensor array 210 includes a plurality of optical sensors arranged in an array shape. For example, the optical sensor array 210 may include a plurality of photodiodes, a plurality of thin film transistors, etc.

The optical sensor array 210 is disposed below the wide-angle emission filter 22 and measures the brightness of emission light such as fluorescence and phosphorescence generated from a sample in the reaction space 240 and passing through the wide-angle emission filter 22. The brightness of emission light measured by the optical sensor array 210 is changed into a light sensing signal and output to the reader system 300.

The temperature sensor 220 is disposed adjacent to the wide-angle emission filter 22 and measures the temperature within the reaction space 240. The temperature measured by the temperature sensor 220 is converted into a temperature signal and output to the first temperature control module 270.

The first temperature control member 230 is disposed below the base substrate and controls the temperature within the reaction space 240 by the control of the first temperature control module 270. In the present embodiment, the first temperature control member 230 may include a heater, a thermoelectric device, or the like. In another embodiment, the first temperature control member 230 may be disposed above the wide-angle emission filter 22, or inside, on a side surface, or above the reaction space 240.

The reaction space 240 is disposed on the wide-angle emission filter 22 and accommodates a sample, thereby performing a PCR operation. The reaction space 240 is formed by a plurality of partitions protruding in a vertical direction from the wide-angle emission filter 22. For example, a plurality of reaction spaces having a small size of about 80 µm to 3 mm may be formed by the plurality of partitions.

The first temperature control module 270 controls the first temperature control member 230 by a signal received from the reader system 300 to control the temperature within the reaction space 240.

The reader system 300 includes a central information processing unit 310, a memory 320, an interface 330, a light source 340, a light source filter 343, a light source driving circuit 345, and a second temperature control module 350. The PCR module 20 is detachably coupled to the reader system 300 and may be removed after being used for a disposable experiment.

The central information processing unit 310 reads out the driving data stored in the memory 320 to drive the second temperature control module 350 and the PCR module 20, and receives light sensing information, temperature information, etc., from the PCR module 20 and stores them in the memory 320 in real time. The central information processing unit 310 calculates the gene amplification amount in real time using the light sensing information, temperature information, etc., received from the PCR module 20 to generate gene amplification amount information. The central information processing unit 310 stores the gene amplification amount information in the memory 320 in real time and transmits it to the interface 330.

The memory 320 is connected to the central information processing unit 310, and drives the second temperature control module 350 and the PCR module 20 using the prestored driving data, and stores the light sensing information, temperature information, etc., in real time. The driving data includes temperature control data, light control data, etc., and may be stored in the memory 320 in the form of data, or may be input from the outside through an input device (not shown) and stored. For example, the memory 320 may include various storage devices such as DDR3, SRAM (Frame), SSD (FLASH), etc.

The interface 330 is connected to the central information processing unit 310 and transmits the gene amplification amount information received in real time from the central information processing unit 310 to the outside or transmits an input signal from the outside to the central information processing unit 310. In the present embodiment, the interface 330 may include a communication system (not shown) such as a Wireless LAN (WLAN), WiFi, Bluetooth, etc., a data interface (not shown) such as a universal serial bus (USB), an inter-integrated circuit (I2C), a universal asynchronous receiver/transmitter (UART), a pulse width modulation (PWM), a low voltage differential signalling (LVDS), a mobile industry processor interface (MIPI), etc., a display device (not shown) such as a liquid crystal display (LCD), an organic light emitting display (OLED), a cathode ray tube (CRT), etc., an input device (not shown) such as a mouse, a keyboard, etc., an output device (not shown) such as a printer, a fax machine, etc.

The light source 340 generates excitation light using the light source driving signal.

The light source filter 343 is disposed below the light source 340 and filters the excitation light generated from the light source 340 to transmit only light having a wavelength of a specific band. The reason for disposing the light source filter 343 in the present embodiment is to minimize noise caused by external light and reduce errors in the optical sensor array 210 due to changes in brightness of the external light.

The light source driving circuit 345 drives the light source 340 using a light source driving signal received from the central information processing unit 310.

The second temperature control module 350 is connected to the central information processing unit 310 and controls the temperature of the PCR module 20 using temperature control data received from the central information processing unit 310.

As described above, according to the present disclosure, the temperature-modifying chuck configured to, in the initial mode, receive temperature-modifying heat, such as cooling heat or heating heat from the Peltier element, and in the temperature control mode, move upward on the shaft to supply temperature-modifying heat to the temperature control surface of the PCR cartridge is disposed in the temperature control module for the PCR device, thereby enabling the temperature of the sample unit to be raised or lowered more quickly.

Although the present disclosure has been described above with reference to examples, it will be understood by those skilled in the art that various modifications and changes may be made to the present disclosure without departing from the spirit and scope of the present disclosure as set forth in the claims below.

<Explanation of Symbols> 110: Motor, 120: Shaft assembly, 121: Rack gear, 122: Sensor actuator, 123: First shaft, 124: Second shaft, 125: First spring damper chuck, 126: Second spring damper chuck 127: Chuck guide member, 130: First support member, 140: Second support member, 150: Peltier element, 160: Temperature-modifying chuck, 162: Lower chuck, 164: Upper chuck, 170: Compression spring chuck, 180: Heat sink assembly, 190: Cooling fan, 20: PCR module, 22: Wide-angle emission filter, 200: Optical sensor substrate, 210: Optical sensor array, 220: Temperature sensor, 230: First temperature control member, 240: Reaction space, 270: First temperature control module, 300: Reader system, 310: Central information processing unit, 320: Memory, 330: Interface, 340: Light source, 343: Light source filter, 345: Light source driving circuit, 350: Second temperature control module

### Industrial Applicability

According to a temperature control module for a PCR device and a PCR device having the same, the temperature-modifying chuck configured to, in an initial mode, receive temperature-modifying heat, such as cooling heat or heating heat from the Peltier element, and in a temperature control mode, move upward on the shaft to supply temperature-modifying heat to the temperature control surface of the PCR cartridge is disposed in the temperature control module for the PCR device, thereby enabling the temperature of the sample unit to be raised or lowered more quickly.

## Claims

1. A temperature control module for a PCR device comprising:
a motor;
a shaft assembly comprising a shaft connected to a rotary axis of the motor so as to move up and down in accordance with a rotation of the motor;
a Peltier element disposed above the shaft assembly; and
a temperature-modifying chuck configured to, in an initial mode, receive temperature-modifying heat from the Peltier element and, in a temperature control mode, move upward on the shaft to supply temperature-modifying heat to a temperature control surface of a PCR cartridge.

2. The temperature control module of claim 1, wherein the temperature-modifying chuck comprises:
a lower chuck having a first area covering the Peltier element; and
an upper chuck having a second area formed to contact the temperature control surface of the PCR cartridge.

3. The temperature control module of claim 2, wherein a first groove is formed on an upper surface of the lower chuck, and a second groove facing the first groove is formed on a lower surface of the upper chuck.

4. The temperature control module of claim 3, further comprising a compression spring chuck disposed in the first groove and the second groove, and configured to perform a buffering function when the upper chuck contacts the temperature control surface of the PCR cartridge.

5. The temperature control module of claim 1, further comprising a heat sink assembly in contact with a heat generating portion of the Peltier element to increase an efficiency of the Peltier element.

6. The temperature control module of claim 5, wherein the heat sink assembly comprises:
one or more heat pipes having one side part in contact with the Peltier element; and
a heat sink connected to the other side part of the heat pipes.

7. The temperature control module of claim 6, further comprising a cooling fan disposed adjacent to the heat sink to cool heat of the heat sink.

8. A PCR device comprising:
a PCR module; and
a temperature control module in contact with the PCR module to raise or lower a temperature of the PCR module, wherein the temperature control module comprises,
a motor;
a shaft assembly comprising a shaft connected to a rotary axis of the motor so as to move up and down in accordance with a rotation of the motor;
a Peltier element disposed above the shaft assembly; and
a temperature-modifying chuck configured to, in an initial mode, receive temperature-modifying heat from the Peltier element and, in a temperature control mode, move upward on the shaft to supply temperature-modifying heat to a temperature control surface of a PCR cartridge.
